# EUROPEAN PATENT SPECIFICATION

(11) **EP 0 843 566 B1**
(45) Date of publication and mention of the grant of the patent: **10.05.2000**
(21) Application number: 95937656.7
(22) Date of filing: 19.10.1995
(51) Int. Cl.: A61M 15/00

(54) **MEDICAMENT CARRIER FOR DRY POWDER INHALATOR AND PROCESS FOR FORMING THE SAME**
ARZNEIMITTELTRÄGER FÜR TROCKENPULVERINHALATOR UND VERFAHREN ZUR HERSTELLUNG DESSELBEN
SUPPORT DE MEDICAMENT POUR INHALATEUR DE POUDRE SECHE ET SON PROCEDE DE FABRICATION

(30) Priority: 21.10.1994 US 328578; 21.10.1994 US 328577
(43) Date of publication of application: 27.05.1998
(60) Divisional application: 99121279.6
(73) Proprietor: GLAXO WELLCOME INC., Research Triangle Park, North Carolina 27709 (US)
(72) Inventor: VAN OORT, Michiel, M., Research Triangle Park, NC 27709 (US)
(74) Representative: Pike, Christopher Gerard
(86) International application number: US9513915
(87) International publication number: WO9612515

(56) References cited:
- WO-A-90/13328
- WO-A-92/00115
- WO-A-94/17679
- WO-A-94/20164
- US-A- 5 101 838

## Description

### Field of the Invention

The present invention relates to a medicament carrier, and more particularly to a carrier containing a dry powder medicament thereon and which is adapted to be positioned within a dry powder inhalator.

### Related Art

Asthma and other respiratory diseases are typically treated by the inhalation of an appropriate medicament for deposition in the lungs to ease patient breathing and increase air capacity. The most widely used treatments for respiratory diseases have been (1) the inhalation of a medicament from a drug solution or suspension in a metered dose aerosol, pressurized inhalator and (2) the inhalation of a powdered drug (generally admixed with an excipient) from a dry powder inhalator. However, in view of recent evidence of the link between chlorofluorocarbon emissions and the deterioration of the earth's atmospheric ozone layer, use of drugs in pressurized inhalators is less desirable and interest in dry powder inhalation systems has substantially increased.

Applicant is presently aware of several different basic methods in use to provide fine particle powders to the respiratory tract without the use of undesirable chlorofluorocarbon propellants. The first method utilizes hard gelatin capsules which contain both a dose of the active material and, in addition, potential adjuvants. The inhalator used by the asthmatic patient for this method comprises a device for perforating or opening the capsule which is then inserted into the inhalator when needed. An air stream generated by the patient on a mouthpiece of the inhalator serves to remove the powder contained within the opened capsule. The empty capsule is then expelled from the inhalator, which is then ready to receive the next capsule. The air stream which passes through the capsule during inhalation acts to remove the powdered medicament from the broken capsule, but it has been found that the air stream created by the patient using this type of inhalator is generally not sufficient in duration to remove all of the contents from the capsule. Dry powder inhalators using this technology are disclosed in a number of prior art references including U.S. Patent Nos. 3,906,950; 4,013,075; 3,807,400; and 3,991,761.

Another type of inhalator device is loaded with a package having a number of blisters which are spaced apart from each other. Each blister contains a fixed quantity of powdered medicament for administration to the patient. As each blister is moved into a predetermined position, it is broken by a suitable means so as to release the powder which is in turn inhaled by the patient. However, it has been found that moisture ingress into the blister pack can cause agglomeration of the prepared medicament therein. Consequently, when the prepared medicament is inhaled by the user, the preferred particle size for greatest efficacy in respiratory disease treatment may not necessarily be achieved. Moreover, the operation of the device requires the use of excipients (e.g., lactose) in order to meter and administer the medicament. This type of inhalation device is disclosed in a number of prior art patent publications including EPO Patent Application Publications Nos. EPO 211595; EPO 455463; and EPO 467172 A1.

Yet another type of dry powder inhalator contains a quantity of medicament therein which is sufficient for multiple doses. A representative example of this type of device is the Draco TURBUHALER® which is disclosed in U.S. Patent Nos. 4,668,218; 4,667,668; and 4,805,811. The inhalator includes a device for withdrawing powdered medicament from the container and for preparing a dose for inhalation. The withdrawal and dose preparation includes a plate having a predetermined thickness and a number of cup-shaped holes therethrough. The plate can be moved by mechanical means from a position where a proportion of the holes are filled with powdered medicament taken from the container to another position in which the holes filled with the medicament are located within a channel. Air flows into the channel as a result of suction provided by the patient on a mouthpiece in fluid communication with the channel so as to remove the powdered medicament from the holes. However, it has been found that when suction is applied to entrain the medicament from one or more holes in the plate, not all of the medicament is entrained in the air flow. Moreover, the TURBUHALER® device is designed to administer large doses and is prone to significant variations in drug delivery.

A fourth dry powder inhalator device is disclosed in German Patent No. 4020571 A1 wherein a velour or velvet- type material loaded with powder is introduced into a jet stream of air. The airstream acts to lift the powder from the velour-like material and to entrain the powder within the airstream which is then in turn inhaled by the patient. One shortcoming of this type of inhalator device is that there is a tendency for the carrier fibers to intermix with the medicament.

A new type of carrier disc for a dry powder inhalator which has recently been proposed is disclosed in PCT Application WO 94/20164 which teaches a screen mesh disc which is impregnated at spaced locations or portions along its circumference with a dose of powdered medicament, such as salmeterol hydroxynapthoate, which can be useful in the treatment of asthma. Since the powdered medicament is impregnated into the interstices of the screen portions, the air impinging upon the screen portions and the powdered medicament during inhalation will cause the medicament to break up so as to aerosol or atomize the medicament. Further, the interstitial deposit of the medicament on the screen portions allows turbulent air to surround each medicament dose and entrain it to assist complete dispensing of the medicament dose from the screen portions into the air-stream. However, the use of the screen disc in the dry powder inhalator device also suffers certain shortcomings including imprecise metering of the powdered medicament since the screen portion interstices are used to meter the medicament. Other shortcomings of the interstitial deposit of the powdered medicament (or impregnation of the medicament) into the screen portions are limitations of dose size to interstitial volume, and the necessity to deaggregate large clusters of medicament present in interstitial voids.

Applicant's present invention avoids many of the problems associated with prior art dry powder inhalators by providing a novel medicament carrier which allows a predetermined and precise dose of the dry powdered medicament to be supplied through the inhalator device upon demand. Moreover, applicant's invention provides much greater flexibility in medicament dose range with a specific carrier screen portion size since the medicament dose is not dependent on the interstitial void volume of the carrier screen portion.

### Summary of the Invention

In accordance with the present invention there is provided a medicament carrier for use in a dry powder inhalator device comprising a carrier having at least one carrier screen portion defining a plurality of interstices therein and loaded with at least one dose of a powdered medicament, wherein the powdered medicament is loaded onto the carrier screen portion surface such that the interstices thereof are at least partially open and free of the powdered medicament.

The present invention also provides a process for forming a medicament carrier to use in a dry powder inhalator device comprising the steps of:
(a) providing a carrier having a plurality of carrier surfaces defining a plurality of interstices therebetween;
(b) applying a predetermined amount of a suspension comprising at least one dose of a powdered medicament to the carrier surfaces of the carrier so that the interstices are at least partially open and free of the suspension; and
(c) evaporating off the suspending agent from the carrier surfaces so as to leave the powdered medicament deposited primarily upon the carrier surfaces of the carrier and not within the interstices thereof.

The present invention further provides a process for forming a medicament carrier to use in a dry powder inhalator device comprising the steps of:
(a) providing a carrier having at least one carrier screen portion defining a plurality of interstices therebetween;
(b) applying a predetermined amount of a suspension comprising at least one dose of a powdered medicament to the surface of the carrier screen portion so that the interstices are at least partially open and free of the suspension; and
(c)evaporating off the suspending agent from the carrier screen portion so as to leave the powdered medicament deposited primarily upon the surfaces of the carrier and not within the interstices thereof.

It is therefore the object of the present invention to provide a medicament carrier for use in a dry powder inhalator which provides for administration of a predetermined precise dosage of the powdered medicament.

It is another object of the present invention to provide a medicament carrier for use in a dry powder inhalator device which provides for the ingested particle size of the powdered medicament dose to be formed for maximum beneficial efficiency.

Some of the objects of the invention being stated, other objects will become evident as the description proceeds, when taken in connection with the accompanying drawings described hereinbelow.

### Brief Description of the Drawings

Figure 1 is a perspective view of a first representative medicament carrier cassette for use in a dry powder inhalator device in accordance with the present invention;
Figure 2 is a perspective view of a second represen-tative medicament carrier cassette for use in a dry powder inhalator device in accordance with the present invention;
Figure 3 is a perspective view of a third representa-tive medicament carrier cassette for use in a dry powder inhalator device in accordance with the present invention;
Figure 4 is a perspective view of an individual medicament carrier such as utilized in the representative cassettes shown in Figures 1-3;
Figure 5 is an exploded perspective view of the medicament carrier shown in Figure 4;
Figure 6 is an exploded vertical cross-sectional view of the medicament carrier shown in Figure 4;
Figure 7 is a schematic view illustrating application of a suspension solution of a powdered medicament being applied to the medicament carrier screen portion shown in Figures 5 and 6;
Figure 8 is an enlarged, perspective view showing loading of the powdered medicament onto the medicament carrier screen portion surface after evaporation of the suspending agent such that the interstices of the medicament carrier screen portion are at least partially open and free of the powdered medicament;
Figure 9 illustrates the medicament carrier of the present invention and the effect thereupon when subjected to an inhalation air pulse;
Figures 10A and 10B show a plan view and an enlargement of a selected portion thereof, respectively, of a medicament carrier screen portion with an accreted powdered medicament loaded thereon; and
Figure 11 illustrates a second embodiment of the medicament carrier of the present invention wherein the carrier comprises two spaced-apart screens having a plurality of substantially spherical substrate elements positioned therebetween which are loaded with powdered medicament and the effect thereupon when subjected to an inhalation air pulse.

### Best Mode For Carrying Out the Invention

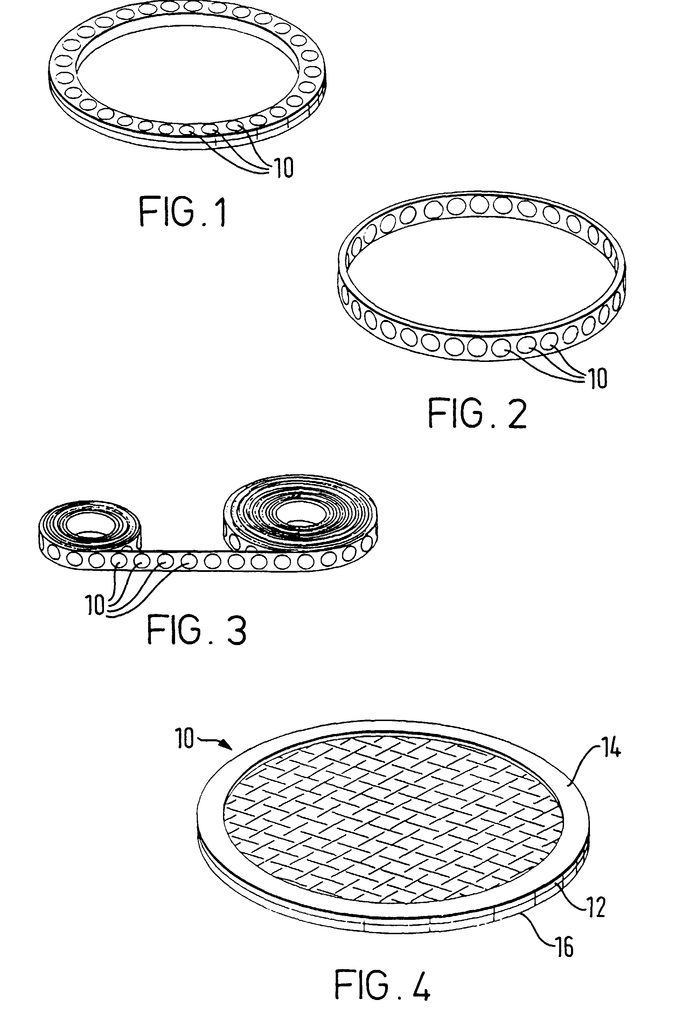

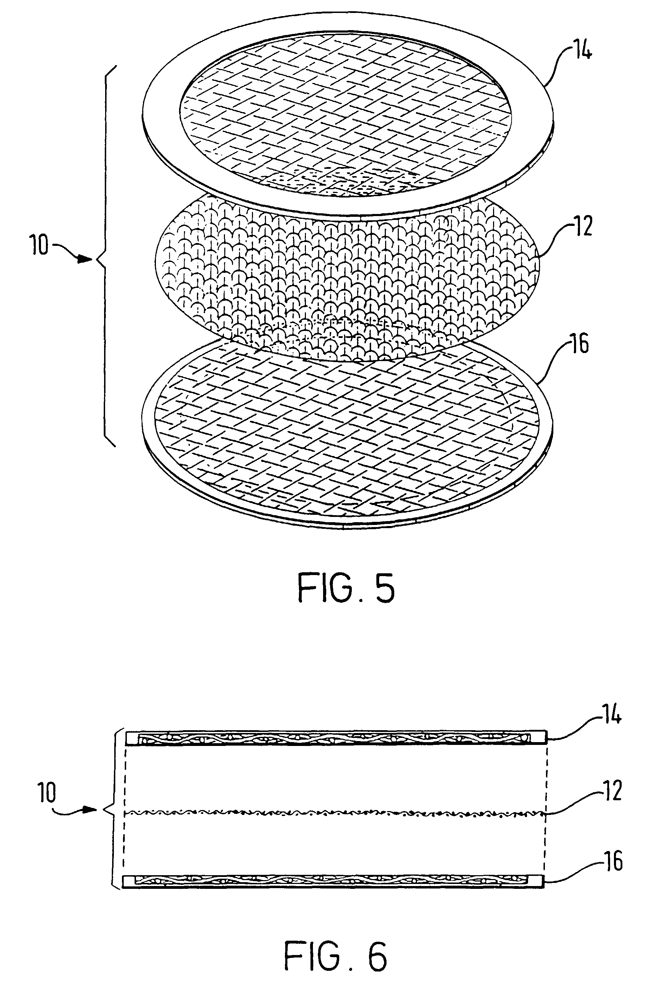

Referring now to Figures 1-11 of the drawings wherein like numerals indicate like elements throughout the several views, three (3) embodiments of medicament carrier cassettes are illustrated in Figures 1-3 which each include a number of spaced-apart medicament carriers **10** therein which form the subject of the instant invention. Powdered medicaments which can be used with the medicament carrier of the present invention for inhalation therapy of systemic absorption via the respiratory tract include, but are not limited to, salbutamol, terbutaline, isoproterenol, metaprotaranol, pirbuterol, salmeterol hydroxynapthoate, fluticasone propionate, budesenide, beclomethasone dipropionate, and triacetonide. Moreover, it is understood that the powdered medicaments may optionally be admixed with excipients such as lactose and glucose.

Referring more specifically to the drawings, medicament carriers **10** are shown in Figures 4-6. Medicament carriers **10**, a plurality of which are included in each of representative medicament carrier cassettes of Figures 1-3, are formed from carrier screen portions **12** which most suitably are secured or sandwiched between enclosure screens **14** and **16,** although screens **14** and **16** would not be required in other embodiments of medicament carriers **10** in accordance with the present invention. It should be appreciated that medicament carrier cassettes of Figures 1-3 are configured so as to be insertable into any suitable breath-activated dry powder inhalator (not shown) such as are well known in the art and that novel medicament carriers **10** of the present invention could be incorporated into many other types of sheets, plates, discs and the like in addition to the three depicted representative cassettes.

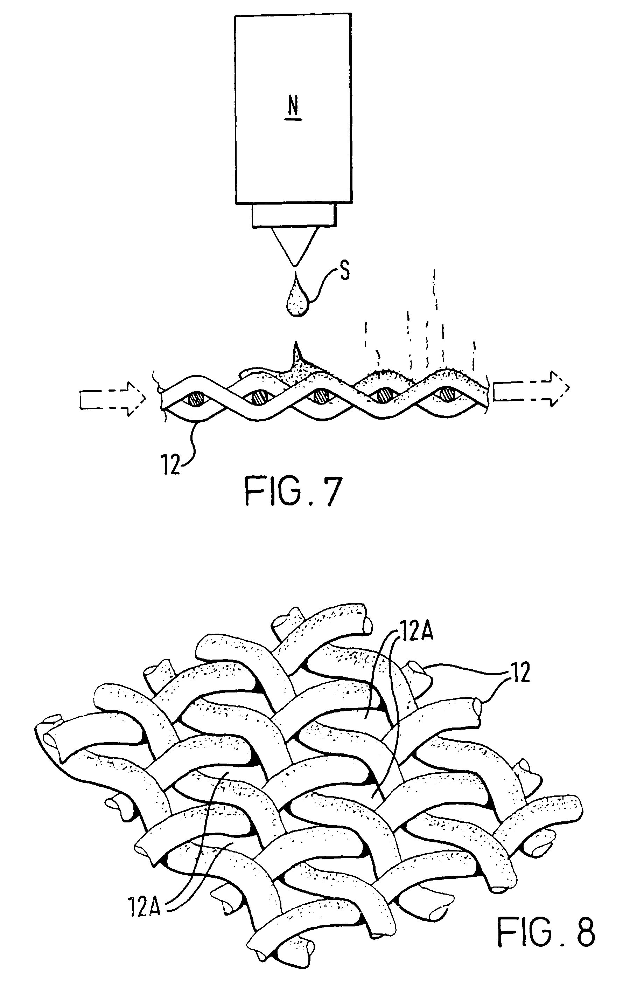

The carrier screen portion **12** can be a non-woven or woven screen formed from natural or synthetic fibers or stamped from a metal blank or photoacid etched from stainless steel or ceramic or formed in any other suitable fashion, so as to provide a plurality of interstices **12A** (see Figure 8) therein. Applicant's invention provides for depositing a prescribed dose of a dry powdered medicament substantially on the surface of carrier screen portion 12 (see Figure 8) and not primarily within the interstices **12A** thereof. The size of the dose depends upon the drug used. For example, a common drug used for asthmatics is salmeterol hydroxynapthoate which is normally dispensed in single doses of about 50 micrograms (µg). Thus, each medicament dose of such a drug could be deposited on the surface of a selected carrier screen portion **12** which is most suitably formed so as to be about 0.15 - 0.64 centimetres (.06 - .25 inches) in diameter in size and to have interstices therein measuring approximately 0.0013 centimetres (.0005 inches) or more in width. As noted hereinbefore, carrier screen portion 12 may be formed in many ways including a non-woven or woven screen formed from natural or synthetic fibers or stamped from a metal blank or photoacid etched from stainless steel or ceramic material.

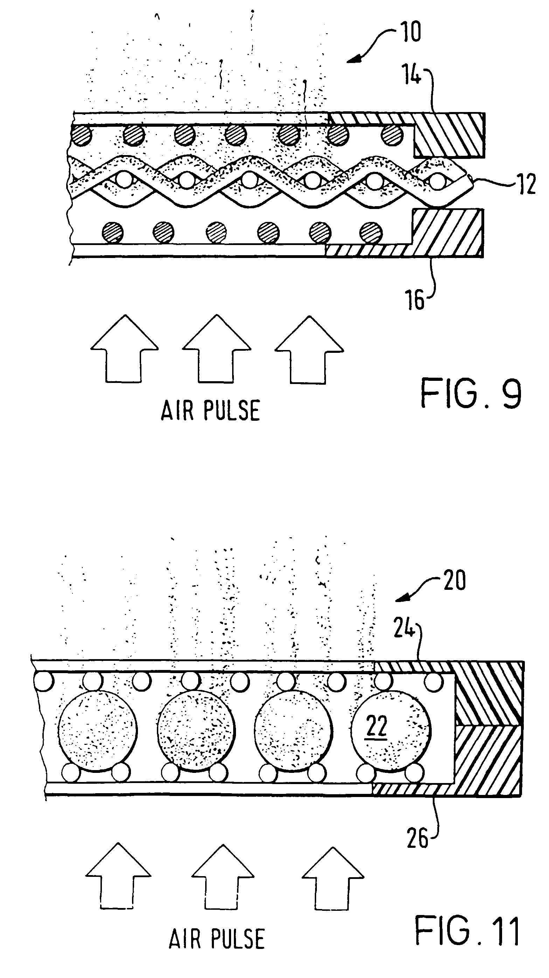

Carrier screen portion **12** is formed with interstices **12A** of approximately 0.0013 centimetres (.0005 inches) or more in width and (optionally) is secured or sandwiched between enclosure screens **14** and **16** so as to form medicament carrier **10**. It is to be understood that medicament carrier **10** could be formed exclusively from carrier screen portion **12** as a matter of design choice in forming medicament carrier **10**. A plurality of medicament carriers **10** are positioned on the perimeter of a medicament carrier cassette such as the rings shown in Figures 1 and 2, respectively, or along the length of a medicament carrier cassette tape such as shown in Figure 3. Optional enclosure screens **14** and **16** each permit access of an external air flow or air pulses through the exposed area of medicament carrier **10** when the carrier is positioned within a suitable dry powder inhalator device (not shown) so that the powdered medicament can be entrained in the air (see Figure 9) which is then inhaled by the patient through the inhalator mouthpiece (not shown) which communicates with the air flow. By suitable mechanical or electromechanical means, medicament carriers **10** within medicament carrier cassettes such as shown in Figures 1-3 are selectively indexed to present a new dose of a powdered medicament to the air flow or air pulse of the inhalator device.

Since the powdered medicament is primarily deposited on the surface of carrier screen portion **12** and spans a significant number of interstices of carrier screen portion **12** (see Figure 8), the number of particles in physical contact with each other is significantly reduced and therefore the amount of energy required to deaggregate the particles into the respirable particle size range is minimized (as opposed, for example, to strictly interstitial deposit of the powdered medicament). The thickness of the layer of powdered medicament on the surface of the elements forming carrier screen portion **12** can be selected so as to minimize the degree of particle-particle contact and/or the size of particle microclusters. The air pulse directed at the dry powdered medicament will serve to sweep the dose of powdered medicament off of carrier screen portion **12**, to suck the dose off of carrier screen portion **12** by virtue of the Bernoulli effect and/or to burst through the dose bridging the interstices.

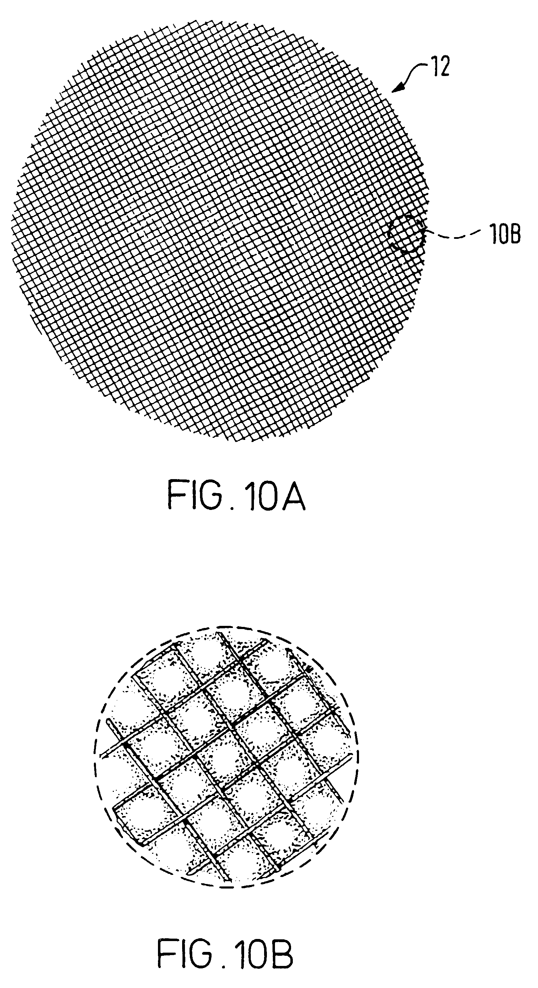

Applicant has discovered that the high shear forces and turbulence experienced by the deposited powdered medicament will result in removal and/or deaggregation of the particles or microclusters of the particles. Thus, each interstice **12A** of carrier screen portion 12 will act as a nozzle or jet if any particles are not directly adhered to the surface of the elements defining carrier screen portion **12** but are accreted thereto (see, for example, Figures 10A and 10B).

Screen **14** (which, as previously noted, is optional and not a required element of the medicament carrier of the invention) is utilized so as to further aid in the deaggregation of the drug particles due to impaction and high shear forces resulting from contact of the powdered medicament (removed by the air flow from carrier screen portion **12** and entrained in the air flow therethrough) with screen **14**. Also, upstream screen **16** serves to modify the air flow so as to maximize turbulence and shear to facilitate deaggregation of the powdered medicament.

An alternative embodiment of applicant's invention contemplates providing medicament carrier **20** (see Figure 11). which does not require deposition of dry powder medicament directly onto the surface of the elements defining carrier screen portions **12**. Alternative embodiment medicament carrier **20** comprises substantially spherical substrate elements **22** formed from materials such as organic or inorganic materials such as metals, polymers or polysaccharides and upon the surfaces of which the dry powdered medicament is deposited. Spherical substrate elements **22** are carried between two screen elements **24** and **26** so as to position spherical substrate elements **22** in the air flow or air pulse through the exposed area of medicament carrier **20** within the air flow channel of an inhalator so that the dry powder medicament can be entrained in the air or aerosolized for inhalation by a patient. Medicament carrier **20** is positioned with an inhalator device (not shown) so that the interstices of screen elements **24** and **26** serve functionally as air jets in order to facilitate deaggregation and removal of the dry powdered medicament from the surfaces of spherical substrate elements **22**.

### Manufacturing Process

Applicant's novel medicament carriers **10** and **20** are most suitably formed by metering small quantities of selected medicaments in the form of a suspension on carrier screen portions **12** or carrier spherical substrate elements **22** and then evaporating off the suspending media. The suspending media should be relatively non-toxic, low or non-flammable, and possess a boiling point near or slightly above room temperature in order to be suitable for a production setting. Applicant has found that perfluoropentane, perfluorohexane, perfluoromethylcyclo-hexane and perfluorocyclohexane serve as good suspending media, although other suspending media are contemplated as being within the scope of the present invention (including fluorinated hydrocarbons and/or hydrocarbons).

The disc, ring or strip or the like that will become a drug cassette (such as shown in Figures 1-3) will be passed into a drug filling zone in the manufacturing process. The manufacturing drug filling zone will consist of an arrangement of spray nozzles or needles **N** (see Figure 7) that will meter out a predetermined amount of a drug as a suspension or solution **S** of the drug onto carrier screen portions **12** or onto spherical substrate elements **22.** Since it is desirable to remove the suspending medium described hereinabove, a sufficient time will be permitted to pass to allow the suspending medium to evaporate. Optionally, heat and/or a small positive or negative air pressure may be applied to carrier screen portions **12** or substantially spherical substrate elements **22** to facilitate evaporation. Thus, the essence of applicant's manufacturing process is the application of the drug onto the surface elements of carrier screen portions **12** and spherical substrate elements **22** so as to leave the interstices therebetween substantially open and free of all drug.

### Experimental Testing

Applicant utilized an extensive survey to select an appropriate suspending medium for dry powder medicament to be applied to the carrier screen portions of the drug carriers of a medicament carrier cassette (e.g., sheet, plate, disc, tape or the like having a plurality of medicament carrier screen portions therein). The selection criteria can include non-flammability, non-toxicity, a boiling point close to room temperature (for high vapor pressure and low energy input to remove the liquid), and low environmental impact. Applicant found perfluoropentane to be a good suspension medium which has significant advantages over many other liquids, although other suspending medium may be used in the practice of the present invention. Micronized salmeterol dry powder medicament may be easily suspended in perfluoropentane, and at refrigerated temperatures the perfluoropentane-salmeterol suspensions appear to be stable for several days.

Applicant studied a number of screen materials for use as the carrier screen portions of the medicament carrier cassette, etc. Physio-chemical properties of the screen material which are important include moisture content, abrasion/heat/ chemical resistance, dimensional stability, physical properties of the screen (such as percent open area, air permeability), thread diameter and weave type. Screen samples for use as carrier screen portions were studied including nylon, polyester, polypropylene and stainless steel, and applicant presently believes stainless steel and non-hygroscopic polymers are preferred screen materials since moisture is a problem with many dry powder medicament formulations. Thus, the screen material should be relatively non-hygroscopic and hydrophobic, and this fact decreases the likelihood of nylon and polyester being suitable screen materials. Polypropylene, ethylene tetrafluoroethylene (ETFE) and E-CTFE are non-hygroscopic and have excellent hydrophobici-ties and thus should be suitable screen materials for forming the carrier screen portions of the medicament carriers of the invention.

Although other types of screens may be used as discussed in some detail hereinabove, stainless steel-type screens were used in the testing to be described in more detail below.

### Testing Results

Applicant's preliminary statistically designed experiments utilized stainless steel carrier screens and investigated the following factors: mesh count (180, 230, 325; same wire diameter, different percentage open area), drug loading (50 µg and 250 µg), dot size (0.25, 0.38, 0.51 cm) (0.1, 0.15, 0.2 inches), air pulse pressure (2.0, 3.5 and 5.0 x 10⁵ Pascals) (2.0, 3.5 and 5.0 atmospheres), air pulse volume (0.1, 0.2 and 0.3 milliliters) and screen configuration (air pulse impacts the drug first -DF, the screen first - SF, and twin screens -TS). Dot size is understood to be the carrier screen diameter.

A 2.5% suspension of fluticasone propionate in perfluoropentane was prepared, and the drug was dispensed or filled onto the screens using an EPPENDORF brand electronic pipette. The particle sizing was accomplished by placing the appropriate screen into the test inhalation dispersing apparatus and firing the dose into an API brand AEROSIZER time-of-flight particle size analyzer.

Summarily, the test results reveal that the best results were obtained by applying the dry powdered medicament to the medicament carrier of the invention using a single screen (no enclosure screens 14 and 16) with high air volume, high air pressure, low drug loading and a small dot size. However, on average the highest particle counts were obtained with a small dot, high drug loading and twin screen configuration.

When the three screen configurations were analyzed separately, applicant discovered that a coarse mesh carrier screen portion worked better for single screen (no enclosure screens 14 and 16) configurations, while a fine mesh performed better for the twin screen (including enclosure screen 14) configuration.

Applicant's analysis performed on only the high air volume/high air pressure measurements confirm the fact that a single screen configuration, low drug loading and a small dot size provided the most favorable particle size distributions, while a twin screen with a high drug loading produced the highest particle counts Additionally, the fine screen mesh tended to provide higher particle counts on the average.

Applicant's test results from which the aforementioned observations were obtained are set forth in Table 1 and Table 2 below.

**TABLE 1**

| | | | |
|---|---|---|---|
| TESTING RESULTS DRUG LOADING = ⁻40 µg AIR PRESSURE = 4.8 x 10⁵ Pa (70psi) AIR VOLUME = 0.3mL | | | |

| Config. | MMAD (µm) | % < 6.3 µm | Counts |
|---|---|---|---|
| DF | 2.3 | 98.5 | 262734 |
| SF | 2.5 | 97.2 | 342523 |
| TS | 6.7 | 49.3 | 752301 |
| NOTE: The results were averaged over screen mesh and dot size | | | |

**TABLE 2**

| | | | |
|---|---|---|---|
| TESTING RESULTS DRUG LOADING = ⁻175 µg AIR PRESSURE = 4.8 x 10⁵ Pa (70 psi) AIR VOLUME = 0.3mL | | | |

| Config. | MMAD (µm) | % < 6.3 µm | Counts |
|---|---|---|---|
| DF | 4.0 | 71.2 | 1090524 |
| SF | 3.5 | 76.2 | 699456 |
| TS | 11.4 | 22.6 | 1704059 |
| NOTE: The results were averaged over screen mesh and dot size | | | |

It will be understood that various details of the invention may be changed without departing from the scope of the invention. Furthermore, the foregoing description is for the purpose of illustration only, and the invention extends to modifications, variations and improvements thereto.

## Claims

1. A medicament carrier (10) for use in a dry powder inhalator device comprising a carrier having at least one carrier screen portion (12) defining a plurality of interstices (12A) therein and loaded with at least one dose of a powdered medicament, characterised in that the powdered medicament is loaded onto the carrier screen portion (12) surface such that the interstices (12A) thereof are at least partially open and free of the powdered medicament.

2. The medicament carrier (10) according to claim 1 wherein said screen portion (12) is formed from a material selected from the group consisting of non-woven or woven screens, stamped metal blanks, photoacid etched stainless steel and photoacid etched ceramic.

3. The medicament carrier (10) according to claim 1 wherein said carrier comprises a plurality of carrier screen portions (12) each loaded with one dose of a powdered medicament.

4. The medicament carrier (10) according to claim 1 wherein the interstices (12A) in said carrier screen mesh portion are at least 0.0013 centimetres (.0005 inches) in width.

5. The medicament carrier (10) according to claim 1 wherein the powdered medicament loaded on said carrier screen portion (12) is selected from the group consisting of salbutamol, terbutaline, isoproterenol, metaprotaranol, pirbuterol, salmeterol hydroxynapthoate, fluticasone propionate, budesenide, beclomethasone dipropionate, and triacetonide.

6. The medicament carrier (10) according to claim 1 wherein at least one screen (14 or 16) is positioned adjacent but spaced apart from said carrier (10) to facilitate deaggregation of the powdered medicament.

7. The medicament carrier (10) according to claim 6 wherein a screen (14 or 16) is positioned adjacent but spaced apart from each side of said carrier to facilitate deaggregation of the powdered medicament.

8. The medicament carrier (10) according to claim 1 wherein the powdered medicament is loaded onto the carrier screen portion (12) surface in a suspension and the suspending agent is evaporated.

9. A process for forming a medicament carrier (10) to use in a dry powder inhalator device comprising the steps of:
(a) providing a carrier having a plurality of carrier surfaces defining a plurality of interstices (12A) therebetween;
(b) characterised by applying a predetermined amount of a suspension comprising at least one dose of a powdered medicament to the carrier surfaces of the carrier (10) so that the interstices (12A) are at least partially open and free of the suspension; and
(c) evaporating off the suspending agent from the carrier surfaces so as to leave the powdered medicament deposited primarily upon the carrier surfaces of the carrier (10) and not within the interstices (12A) thereof.

10. The process according to claim 9 wherein said carrier surfaces comprises a carrier screen portion (12).

11. The process according to claim 9 wherein said carrier surfaces comprises a plurality of spherical substrate elements.

12. The process according to claim 9 wherein said suspension comprises a powdered medicament selected from the group consisting of salbutamol, terbutaline, isoproterenol, metaprotaranol, pirbuterol, salmeterol hydroxynapthoate, fluticasone propionate, budesenide, beclomethasone dipropionate, and triacetonide.

13. The process according to claim 9 wherein the suspending agent is selected from the group consisting of perfluoropentane, perfluorohexane, perfluoromethylcyclo-hexane and perfluorocyclohexane.

14. The process according to claim 9 wherein the suspending agent comprises fluorinated hydrocarbons and/or hydrocarbons.

15. A process for forming a medicament carrier to use in a dry powder inhalator device comprising the steps of:
(a) providing a carrier (10) having at least one carrier screen portion (12) defining a plurality of interstices (12A) therebetween;
(b) characterised by applying a predetermined amount of a suspension comprising at least one dose of a powdered medicament to the surface of the carrier screen portion (12) so that the interstices (12A) are at least partially open and free of the suspension; and
(c) evaporating off the suspending agent from the carrier screen portion (12) so as to leave the powdered medicament deposited primarily upon the surfaces of the carrier (10) and not within the interstices (12A) thereof.

16. The process according to claim 15 wherein said suspension comprises a powdered medicament selected from the group consisting of salbutamol, terbutaline, isoproterenol, metaprotaranol, pirbuterol, salmeterol hydroxynapthoate, fluticasone propionate, budesenide, beclomethasone dipropionate, and triacetonide.

17. The process according to claim 15 wherein the suspending agent is selected from the group consisting of perfluoropentane, perfluorohexane, perfluoromethylcyclo-hexane and perfluorocyclohexane.

18. The process according to claim 15 wherein the suspending agent comprises fluorinated hydrocarbons and/or hydrocarbons.

## Patentansprüche

1. Medikamentträger (10) zur Verwendung in einer Trockenpulver-Inhalatorvorrichtung, umfassend einen Träger mit wenigstens einem Träger-Gitterteil (12), der eine Anzahl von Lücken (12A) darin definiert und mit wenigstens einer Dosis eines pulverförmigen Medikaments beladen ist,
dadurch **gekennzeichnet,** daß
das pulverförmige Medikament auf die Oberfläche des Träger-Gitterteils (12) so geladen ist, daß deren Lücken (12A) wenigstens teilweise offen und frei von pulverförmigem Medikament sind.

2. Medikamentträger (10) gemäß Anspruch 1, worin der Gitterteil (12) aus einem Material gebildet ist, ausgewählt aus der Gruppe, die aus Vlies- oder Webgittern, ausgestanzten Metallplatten, photogeätztem rostfreiem Stahl und photogeätzter Keramik besteht.

3. Medikamentträger (10) gemäß Anspruch 1, worin der Träger eine Anzahl von Träger-Gitterteilen (12) umfaßt, die jeweils mit einer Dosis eines pulverförmigen Medikaments beladen sind.

4. Medikamentträger (10) gemäß Anspruch 1, worin die Lücken (12A) im Träger-Gittermaschenteil eine Weite von wenigstens 0,0013 cm (0,0005 Zoll) aufweisen.

5. Medikamentträger (10) gemäß Anspruch 1, worin das auf den Träger-Gitterteil (12) geladene pulverförmige Medikament ausgewählt ist aus der Gruppe, die aus Salbutamol, Terbutalin, Isoproterenol, Metaprotaranol, Pirbuterol, Salmeterolhydroxynaphthoat, Fluticasonpropionat, Budesenid, Beclomethasondipropionat und Triacetonid besteht.

6. Medikamentträger (10) gemäß Anspruch 1, worin wenigstens ein Gitter (14 oder 16) benachbart aber mit Zwischenraum zum Träger (10) positioniert ist, um die Entklumpung des pulverförmigen Medikaments zu erleichtern.

7. Medikamentträger (10) gemäß Anspruch 6, worin ein Gitter (14 oder 16) benachbart aber mit Zwischenraum zu jeder Seite des Trägers positioniert ist, um die Entklumpung des pulverförmigen Medikaments zu erleichtern.

8. Medikamentträger (10) gemäß Anspruch 1, worin das pulverförmige Medikament auf die Oberfläche des Träger-Gitterteils (12) in einer Suspension geladen ist und das Suspendiermittel verdampft ist.

9. Verfahren zur Bildung eines Medikamentträgers (10) zur Verwendung in einer Trockenpulver-Inhalatorvorrichtung, umfassend die Schritte:
(a) Bereitstellen eines Trägers mit einer Anzahl von Trägeroberflächen, die eine Anzahl von Lücken (12A) darin definieren;
**gekennzeichnet** durch
(b) Aufbringen einer festgelegten Menge einer Suspension, die wenigstens eine Dosis eines pulverförmigen Medikaments umfaßt, auf die Trägeroberflächen des Trägers (10), so daß die Lücken (12A) wenigstens teilweise offen und frei von der Suspension sind; und
(c) Verdampfen des Suspendiermittels von den Trägeroberflächen, um das pulverförmige Medikament primär auf den Trägeroberflächen des Trägers (10) und nicht innerhalb der Lücken (12A) davon abgeschieden zurückzulassen.

10. Verfahren gemäß Anspruch 9, worin die Trägeroberflächen einen Träger-Gitterteil (12) umfassen.

11. Verfahren gemäß Anspruch 9, worin die Trägeroberflächen eine Anzahl von sphärischen Substratelementen umfassen.

12. Verfahren gemäß Anspruch 9, worin die Suspension ein pulverförmiges Medikament umfaßt, ausgewählt aus der Gruppe, die aus Salbutamol, Terbutalin, Isoproterenol, Metaprotaranol, Pirbuterol, Salmeterolhydroxynaphthoat, Fluticasonpropionat, Budesenid, Beclomethasondipropionat und Triacetonid besteht.

13. Verfahren gemäß Anspruch 9, worin das Suspendiermittel ausgewählt ist aus der Gruppe, die aus Perfluorpentan, Perfluorhexan, Perfluormethylcyclohexan und Perfluorcyclohexan besteht.

14. Verfahren gemäß Anspruch 9, worin das Suspendiermittel fluorierte Kohlenwasserstoffe und/oder Kohlenwasserstoffe umfaßt.

15. Verfahren zur Bildung eines Medikamentträgers zur Verwendung in einer Trockenpulver-Inhalatorvorrichtung, umfassend die Schritte:
(a) Bereitstellen eines Trägers (10) mit wenigstens einem Träger-Gitterteil (12), der eine Anzahl von Lücken (12A) darin definiert;
gekennzeichnet durch
(b) Aufbringen einer festgelegten Menge einer Suspension, die wenigstens eine Dosis eines pulverförmigen Medikaments umfaßt, auf den Träger-Gitterteil (12), so daß die Lücken (12A) wenigstens teilweise offen und frei von der Suspension sind; und
(c) Verdampfen des Suspendiermittels vom Träger-Gitterteil, um das primär auf den Oberflächen des Trägers (10) und nicht innerhalb der Lücken (12A) davon abgeschieden zurückzulassen.

16. Verfahren gemäß Anspruch 15, worin die Suspension ein pulverförmiges Medikament umfaßt, ausgewählt aus der Gruppe, die aus Salbutamol, Terbutalin, Isoproterenol, Metaprotaranol, Pirbuterol, Salmeterolhydroxynaphthoat, Fluticasonpropionat, Budesenid, Beclomethasondipropionat und Triacetonid besteht.

17. Verfahren gemäß Anspruch 15, worin das Suspendiermittel ausgewählt ist aus der Gruppe, die aus Perfluorpentan, Perfluorhexan, Perfluormethylcyclohexan und Perfluorcyclohexan besteht.

18. Verfahren gemäß Anspruch 15, worin das Suspendiermittel fluorierte Kohlenwasserstoffe und/oder Kohlenwasserstoffe umfaßt.

## Revendications

1. Support de médicament (10) destiné à être utilisé dans un dispositif inhalateur de poudre sèche comprenant un support comportant au moins une partie de tamis de support (12) définissant une pluralité d'interstices (12a) et chargée d'au moins une dose d'un médicament pulvérulent, caractérisé en ce que le médicament pulvérulent est chargé sur la surface de la partie de tamis de support (12) de telle sorte que les interstices (12A) de celle-ci soient au moins partiellement ouverts et sans médicament pulvérulent.

2. Support de médicament (10) suivant la revendication 1, dans lequel la partie de tamis (12) est formée d'une matière choisie dans le groupe comprenant les tamis non tissés ou tissés, les pièces brutes métalliques estampées, l'acide inoxydable attaqué par photoacide et les matériaux céramiques attaqués par photoacide.

3. Support de médicament (10) suivant la revendication 1, dans lequel ledit support comprend une pluralité de parties de tamis de support (12) chargées chacune d'une dose d'un médicament pulvérulent.

4. Support de médicament (10) suivant la revendication 1, dans lequel les interstices (12A) de la partie maillée ou de tamis de support ont au moins 0,0013 cm (0,0005 pouce) de largeur.

5. Support de médicament (10) suivant la revendication 1, dans lequel le médicament pulvérulent chargé sur la partie de tamis de support (12) est choisi dans le groupe comprenant le salbutamol, la terbutaline, l'isoprotérénol, le métaprotaranol, le pirbutérol, l'hydroxynapthoate de salmétérol, le propionate de fluticasone, le budésénide, le dipropionate de béclométhasone et le triacétonide.

6. Support de médicament (10) suivant la revendication 1, dans lequel au moins un tamis (14 ou 16) est positionné de façon adjacente mais espacée dudit support (10) pour faciliter la désagrégation du médicament pulvérulent.

7. Support de médicament (10) suivant la revendication 6, dans lequel un tamis (14 ou 16) est positionné de façon adjacente mais espacée de chaque face dudit support pour faciliter la désagrégation du médicament pulvérulent.

8. Support de médicament (10) suivant la revendication 1, dans lequel le médicament pulvérulent est chargé sur la surface de la partie de tamis de support (12) dans une suspension et l'agent de mise en suspension est évaporé.

9. Procédé de formation d'un support de médicament (10) destiné à être utilisé dans un dispositif inhalateur de poudre sèche comprenant les étapes suivantes :
(a) la présence d'un support comportant une pluralité de surfaces de support y définissant une pluralité d'interstices (12A);
(b) caractérisé par l'application d'une quantité prédéterminée d'une suspension comprenant au moins une dose d'un médicament pulvérulent aux surfaces de support du support (10) de telle sorte que les interstices (12A) soient au moins partiellement ouverts et exempts de la suspension; et
(c) l'évacuation par évaporation de l'agent de mise en suspension des surfaces de support de manière à laisser le médicament pulvérulent déposé principalement sur les surfaces de support du support (10) et non dans les interstices (12A) de celles-ci.

10. Procédé suivant la revendication 9, dans lequel les surfaces de support comprennent une partie de tamis de support (12).

11. Procédé suivant la revendication 9, dans lequel les surfaces de support comprennent une pluralité d'éléments de substrat sphériques.

12. Procédé suivant la revendication 9, dans lequel ladite suspension comprend un médicament pulvérulent choisi dans le groupe comprenant le salbutamol, la terbutaline, l'isoprotérénol, le métaprotaranol, le pirbutérol, l'hydroxynapthoate de salmétérol, le propionate de fluticasone, le budésénide, le dipropionate de béclométhasone et le triacétonide.

13. Procédé suivant la revendication 9, dans lequel l'agent de mise en suspension est choisi dans le groupe comprenant le perfluoropentane, le perfluorohexane, le perfluorométhylcyclohexane et le perfluorocyclohexane.

14. Procédé suivant la revendication 9, dans lequel l'agent de mise en suspension comprend des hydrocarbures fluorés et/ou des hydrocarbures.

15. Procédé de formation d'un support de médicament destiné à être utilisé dans un dispositif inhalateur de poudre sèche comprenant les étapes suivantes :
(a) la présence d'un support (10) comportant au moins une partie de tamis de support (12) y définissant une pluralité d'interstices (12A);
(b) caractérisé par l'application d'une quantité prédéterminée d'une suspension comprenant au moins une dose d'un médicament pulvérulent à la surface de la partie de tamis de support (12) de telle sorte que les interstices (12A) soient au moins partiellement ouverts et exempts de la suspension; et
(c) l'évacuation par évaporation de l'agent de mise en suspension de la partie de tamis de support (12) de manière à laisser le médicament pulvérulent déposé principalement sur les surfaces du support (10) et non dans les interstices (12A) de celles-ci.

16. Procédé suivant la revendication 15, dans lequel ladite suspension comprend un médicament pulvérulent choisi dans le groupe comprenant le salbutamol, la terbutaline, l'isoprotérénol, le métaprotaranol, le pirbutérol, l'hydroxynapthoate de salmétérol, le propionate de fluticasone, le budésénide, le dipropionate de béclométhasone et le triacétonide.

17. Procédé suivant la revendication 15, dans lequel l'agent de mise en suspension est choisi dans le groupe comprenant le perfluoropentane, le perfluorohexane, le perfluorométhylcyclohexane et le perfluorocyclohexane.

18. Procédé suivant la revendication 15, dans lequel l'agent de mise en suspension comprend des hydrocarbures fluorés et/ou des hydrocarbures.
